# EUROPEAN PATENT APPLICATION

(11) **EP 4 818 316 A1**
(43) Date of publication of application: **07.10.2026**
(21) Application number: 25904832.0
(22) Date of filing: 01.08.2025
(51) Int. Cl.: C12N 9/12, C12N 15/77, C12P 13/14, C12P 13/10

(54) **ASPARTATE KINASE MUTANT AND AMINO ACID PRODUCTION METHOD USING SAME**

(30) Priority: 12.12.2024 KR 20240185046
(71) Applicant: CJ Cheiljedang Corporation, Seoul 04560 (KR)
(72) Inventor: LEE, Ji Yeon, Seoul 04560 (KR); PARK, So-Yeon, Seoul 04560 (KR); YOON, Byoung Hoon, Seoul 04560 (KR); KIM, Ye-Eun, Seoul 04560 (KR)
(74) Representative: Meissner Bolte Partnerschaft mbB
(86) International application number: PCT/KR2025/011527
(87) International publication number: WO 2026/127263

(57) **Abstract**

The present disclosure relates to a modified aspartate kinase and a method for producing amino acids using the same.

## Description

### [Technical Field]

The present disclosure relates to a modified aspartate kinase, an amino acid-producing microorganism modified to express the modified aspartate kinase, and a method for producing amino acids using the same.

### [Background Art]

Glutamate is one of the proteinogenic amino acids widely found in plants, animals, and microorganisms, and is metabolized in bodily organs by being converted into ornithine (L-ornithine), citrulline (L-citrulline), arginine (L-arginine), *etc*.

Since ornithine is effective in promoting muscle synthesis and reducing body fat, it is used as a nutritional supplement and also as a pharmaceutical for improving liver cirrhosis and hepatic dysfunction. Citrulline is known to have physiologically active effects such as promotion of ammonia metabolism, improvement of blood flow through vasodilation, reduction of blood pressure, neurotransmission, enhancement of immunity, and scavenging of reactive oxygen species. In addition, arginine is used for medicinal purposes such as liver function promoters, brain function promoters, and comprehensive amino acid preparations, and is also used for food purposes such as fish cake additives, health beverage additives, and salt substitutes for hypertensive patients.

Microorganisms of the genus *Corynebacterium*, particularly *Corynebacterium glutamicum*, are Gram-positive microorganisms widely used for the production of amino acids. For amino acid production, target substance-specific approaches have mainly been used in strains of the genus *Corynebacterium*, such as increasing the expression level of genes encoding enzymes involved in amino acid biosynthesis or eliminating genes not required for amino acid biosynthesis (US 9644009 B2).

### [Disclosure]

### [Technical Problem]

The present disclosure provides a modified aspartate kinase, wherein an amino acid corresponding to position 250 and/or position 258 in the amino acid sequence of SEQ ID NO: 1 is substituted with another amino acid.

### [Technical Solution]

One object of the present disclosure is to provide a modified aspartate kinase, wherein an amino acid corresponding to position 250 and/or position 258 in the amino acid sequence of SEQ ID NO: 1 is substituted with another amino acid.

Another object of the present disclosure is to provide a polynucleotide encoding the modified aspartate kinase of the present disclosure.

Still another object of the present disclosure is to provide a microorganism comprising the modified aspartate kinase of the present disclosure, a polynucleotide encoding the same, or a vector comprising the polynucleotide.

Still another object of the present disclosure is to provide a method for producing an amino acid comprising culturing, in a medium, a microorganism comprising the modified aspartate kinase of the present disclosure, a polynucleotide encoding the same, or a vector comprising the polynucleotide.

Still another object of the present disclosure is to provide a composition for producing an amino acid, comprising the modified aspartate kinase of the present disclosure; a polynucleotide encoding the same; a microorganism comprising the modified aspartate kinase, a polynucleotide encoding the same, or a vector comprising the polynucleotide; a culture of the microorganism; or a combination of two or more thereof.

Still another object of the present disclosure is to provide use of the modified aspartate kinase of the present disclosure; a polynucleotide encoding the same; or a microorganism comprising the modified aspartate kinase, a polynucleotide encoding the same, or a vector comprising the polynucleotide, for amino acid production.

### [Advantageous Effects]

Culturing a microorganism that produces amino acids using the modified aspartate kinase of the present disclosure enables production of amino acids including ornithine, citrulline, and arginine at a higher yield compared to a microorganism having the conventional wild-type aspartate kinase.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific descriptions set forth below. Additionally, a number of papers and patent documents have been cited throughout the present specification. The content of the cited papers and patent documents is incorporated herein by reference in its entirety to describe the level of the technical field to which the present disclosure belongs and the contents of the present disclosure more clearly.

One aspect of the present disclosure provides a modified aspartate kinase, wherein an amino acid corresponding to position 250 and/or position 258 in the amino acid sequence of SEQ ID NO: 1 is substituted with another amino acid.

The modified aspartate kinase of the present disclosure may be one in which an amino acid corresponding to position 250 and/or position 258 in the amino acid sequence of SEQ ID NO: 1 is substituted with an amino acid different from the amino acid before substitution. Alternatively, the modified aspartate kinase may be a modified aspartate kinase in which an amino acid is substituted with an amino acid different from the amino acid before substitution, but is not limited thereto.

The amino acid before substitution corresponding to position 250 in the amino acid sequence of SEQ ID NO: 1 may be valine (Val or V), and the amino acid before substitution corresponding to position 258 may be valine (Val or V).

Specifically, the modified aspartate kinase may be one in which the amino acid corresponding to position 250 and/or position 258 in the amino acid sequence of SEQ ID NO: 1 is substituted with an amino acid selected from the group consisting of methionine, serine, threonine, asparagine, cysteine, histidine, lysine, aspartic acid, alanine, tyrosine, leucine, glutamine, glycine, proline, glutamic acid, arginine, isoleucine, phenylalanine, and tryptophan, but is not limited thereto.

More specifically, the modified aspartate kinase may be one in which the amino acid corresponding to position 250 and/or position 258 in the amino acid sequence of SEQ ID NO: 1 is substituted with an amino acid selected from the group consisting of glycine, alanine, leucine, isoleucine, methionine, phenylalanine, tryptophan, proline, serine, threonine, cysteine, tyrosine, asparagine, and glutamine.

More specifically, the modified aspartate kinase may be one in which the amino acid corresponding to position 250 and/or position 258 in the amino acid sequence of SEQ ID NO: 1 is substituted with an amino acid selected from the group consisting of glycine, alanine, leucine, isoleucine, methionine, phenylalanine, tryptophan, proline, and cysteine.

More specifically, the modified aspartate kinase may be one in which the amino acid corresponding to position 250 and/or position 258 in the amino acid sequence of SEQ ID NO: 1 is substituted with leucine or isoleucine.

More specifically, the modified aspartate kinase may be one in which the amino acid corresponding to position 250 in the amino acid sequence of SEQ ID NO: 1 is substituted with leucine; the amino acid corresponding to position 258 in the amino acid sequence of SEQ ID NO: 1 is substituted with isoleucine; or a combination thereof.

The protein subject to mutation introduction of the present disclosure may be a protein having aspartate kinase activity. Specifically, the protein may comprise the amino acid sequence of SEQ ID NO: 1 and have aspartate kinase activity, but is not limited thereto.

Furthermore, an addition of meaningless sequences before or after the amino acid sequence of SEQ ID NO: 1, naturally occurring mutations, or silent mutations thereof are not excluded, and any protein having the same or corresponding activity as a protein comprising the amino acid sequence of SEQ ID NO: 1 may constitute a protein subject to mutation introduction of the present disclosure. For example, the protein subject to mutation introduction of the present disclosure may be a protein composed of the amino acid sequence of SEQ ID NO: 1, or an amino acid sequence having 70%, 75%, 80%, 85%, 90%, 95%, 96%, 97%, 98%, 99%, or 99.1%, or more homology or identity thereto. Additionally, it is apparent that any protein having an amino acid sequence, in which part of the sequence is deleted, modified, substituted, or added, also falls within the scope of the protein subject to mutation introduction of the present disclosure, as long as the amino acid sequence has such a homology or identity and exhibits an equivalent efficacy to the protein.

The modified aspartate kinase of the present disclosure may comprise an amino acid sequence in which the amino acid corresponding to position 250 in the amino acid sequence of SEQ ID NO: 1 is substituted with another amino acid, and which has 70%, 75%, 80%, 85%, 90%, 95%, 96%, 97%, 98%, 99%, 99.5%, 99.7%, or 99.9%, or more homology or identity to the amino acid sequence of SEQ ID NO: 1.

The modified aspartate kinase of the present disclosure may comprise an amino acid sequence in which the amino acid corresponding to position 250 in the amino acid sequence of SEQ ID NO: 1 is an amino acid other than valine, and which has 70%, 75%, 80%, 85%, 90%, 95%, 96%, 97%, 98%, 99%, 99.5%, 99.7%, or 99.9%, or more homology or identity to the amino acid sequence of SEQ ID NO: 1. Additionally, it is apparent that any modified aspartate kinase having an amino acid sequence, in which part of the sequence is deleted, modified, substituted, conservatively substituted, or added, also falls within the scope of the present disclosure, as long as the amino acid sequence has such a homology or identity and exhibits an equivalent efficacy to the modified aspartate kinase of the present disclosure.

The modified aspartate kinase of the present disclosure may comprise an amino acid sequence in which the amino acid corresponding to position 258 in the amino acid sequence of SEQ ID NO: 1 is substituted with another amino acid, and which has 70%, 75%, 80%, 85%, 90%, 95%, 96%, 97%, 98%, 99%, 99.5%, 99.7%, or 99.9%, or more homology or identity to the amino acid sequence of SEQ ID NO: 1.

The modified aspartate kinase of the present disclosure may comprise an amino acid sequence in which the amino acid corresponding to position 258 in the amino acid sequence of SEQ ID NO: 1 is an amino acid other than valine, and which has 70%, 75%, 80%, 85%, 90%, 95%, 96%, 97%, 98%, 99%, 99.5%, 99.7%, or 99.9%, or more homology or identity to the amino acid sequence of SEQ ID NO: 1. Additionally, it is apparent that any modified aspartate kinase having an amino acid sequence, in which part of the sequence is deleted, modified, substituted, conservatively substituted, or added, also falls within the scope of the present disclosure, as long as the amino acid sequence has such a homology or identity and exhibits an equivalent efficacy to the modified aspartate kinase of the present disclosure.

For example, this includes cases of addition or deletion of a sequence that does not alter the function of the modified aspartate kinase of the present disclosure, naturally occurring mutations, silent mutations, or conservative substitutions at the N-terminus, the C-terminus, and/or within the amino acid sequence.

As used herein, the term "conservative substitution" refers to the substitution of an amino acid with another amino acid having similar structural and/or chemical properties. Such an amino acid substitution may generally occur based on similarity in polarity, charge, solubility, hydrophobicity, hydrophilicity, and/or amphipathic nature of the residues. Typically, conservative substitution may have little or no effect on the activity of a protein or polypeptide.

For example, among the amino acids having an electrically charged side chain (electrically charged amino acids), positively charged (basic) amino acids include arginine, lysine, and histidine, and negatively charged (acidic) amino acids include glutamic acid and aspartic acid; and amino acids having an uncharged side chain can be classified as including glycine, alanine, valine, leucine, isoleucine, methionine, phenylalanine, tryptophan, proline, serine, threonine, cysteine, tyrosine, asparagine, and glutamine.

As used herein, the terms "modified protein" or "variant" refer to a polypeptide having at least one amino acid different from the amino acid sequence of the protein before mutation by conservative substitution and/or modification such that the functions and properties of the protein are retained. Such a variant may generally be identified by modifying at least one amino acid in the amino acid sequence of the polypeptide and evaluating the properties of the modified polypeptide. That is, the ability of the variant may be enhanced, unchanged, or reduced relative to the polypeptide before mutation. Additionally, some variants may include variants in which at least one portion, such as an N-terminal leader sequence or transmembrane domain, has been removed. Other variants may include variants in which a region has been partially removed from the N- and/or C-terminus of a mature protein. The term "modified protein" may be used interchangeably with terms such as modification, modified polypeptide, mutant, mutein, and divergent, but is not limited thereto, as long as the terms are used to indicate a mutation.

Additionally, the variant may also comprise deletion or addition of amino acids that have minimal influence on the properties and secondary structure of the polypeptide. For example, a signal (or leader) sequence involved in the co-translational or post-translational translocation of proteins may be conjugated to the N-terminus of the variant. Further, the variant may also be conjugated with another sequence or linker for identification, purification, or synthesis.

As used herein, the term "homology" or "identity" refers to a degree of similarity between two given amino acid sequences or nucleotide sequences, and may be expressed as a percentage. The terms homology and identity may often be used interchangeably.

The sequence homology or identity of conserved polynucleotides or polypeptides may be determined by a standard alignment algorithm, and default gap penalties established by the program used may be applied together. Substantially, homologous or identical sequences are generally capable of hybridizing with the whole sequence or a part of the sequence under moderately or highly stringent conditions. It is apparent that hybridization also includes hybridization with a polynucleotide containing a general codon or a codon considering codon degeneracy in the polynucleotide.

Whether any two polynucleotide or polypeptide sequences have homology, similarity, or identity may be determined by, for example, a known computer algorithm such as the "FASTA" program using default parameters as in Pearson et al. (1988) [Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al., Nucleic Acids Research 12: 387 (1984)), BLASTP, BLASTN, and FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.] (1988) SIAM J Applied Math 48: 1073)). For example, homology, similarity, or identity may be determined using BLAST of the National Center for Biotechnology Information, or ClustalW.

The homology, similarity, or identity between polynucleotides or polypeptides may be determined by, for example, comparing sequence information using a GAP computer program, such as Needleman et al., (1970), J Mol Biol. 48:443, as described in, for example, Smith and Waterman, Adv. Appl. Math (1981) 2:482. Briefly, a GAP program may be defined as the value acquired by dividing the number of similarly aligned symbols (namely, nucleotides or amino acids) by the total number of symbols in the shorter of the two sequences. The default parameters for the GAP program may comprise: (1) a binary comparison matrix (comprising values of 1 for identity and 0 for non-identity) and a weighted comparison matrix of Gribskov et al. (1986) Nucl. Acids Res. 14: 6745 (or EDNAFULL (EMBOSS version of NCBI NUC4.4) substitution matrix), as disclosed in Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap opening penalty of 10, gap extension penalty of 0.5); and (3) no penalty for end gaps.

In one example of the present disclosure, the modified aspartate kinase of the present disclosure may have an improved amino acid-producing ability compared to a wild-type polypeptide having aspartate kinase activity.

In another example, the modified aspartate kinase of the present disclosure may have an activity that increases amino acid-producing ability compared to a wild-type polypeptide having aspartate kinase activity.

As used in the present disclosure, the term "aspartate kinase (Aspartokinase, AK, LysC)" refers to an enzyme having an activity that catalyzes the phosphorylation of the amino acid aspartate in microorganisms, and may be used interchangeably with LysC. The amino acid sequence of LysC can be obtained from publicly known databases such as NCBI GenBank.

In one example, LysC of the present disclosure may be derived from a microorganism, specifically from a prokaryotic or eukaryotic microorganism, and more specifically from microorganisms of the genus *Corynebacterium*, *etc*.; however, it is apparent that proteins of various origins having an activity that catalyzes the phosphorylation of aspartate are included.

In another example, the LysC protein may be NCgl0247 (cg0306) derived from a microorganism of the genus *Corynebacterium*; however, it is apparent that proteins of various origins having aspartate kinase activity are included.

In the present disclosure, the amino acid before modification corresponding to position 250 and/or position 258 of SEQ ID NO: 1 in the amino acid sequence before modification of the LysC protein subject to mutation may be valine (V).

The modified aspartate kinase of the present disclosure may be one in which an amino acid corresponding to position 250 and/or position 258 in the amino acid sequence of SEQ ID NO: 1 is substituted with an amino acid different from the amino acid before substitution.

In one example, the modified aspartate kinase may be one in which the amino acid corresponding to position 250 and/or position 258 in the amino acid sequence of SEQ ID NO: 1 is substituted with at least one amino acid selected from the group consisting of methionine, serine, threonine, asparagine, cysteine, histidine, lysine, aspartic acid, alanine, tyrosine, leucine, glutamine, glycine, proline, glutamic acid, arginine, isoleucine, phenylalanine, and tryptophan. In another example, the modified aspartate kinase may be one in which an amino acid corresponding to position 250 and/or position 258 in the amino acid sequence of SEQ ID NO: 1 is substituted with an amino acid other than an aromatic amino acid. In another example, the modified aspartate kinase may be one in which an amino acid corresponding to position 250 and/or position 258 in the amino acid sequence of SEQ ID NO: 1 is substituted with an amino acid having a nonpolar, polar, or charged side chain.

In another example, the modified aspartate kinase of the present disclosure may be one in which the amino acid corresponding to position 250 in the amino acid sequence of SEQ ID NO: 1 is substituted with leucine; the amino acid corresponding to position 258 in the amino acid sequence of SEQ ID NO: 1 is substituted with isoleucine; or a combination thereof.

In another example, the modified aspartate kinase of the present disclosure may have, comprise, consist of, or essentially consist of the amino acid sequence represented by SEQ ID NO: 3, SEQ ID NO: 5, or SEQ ID NO: 7.

As used herein, the term "corresponding to" refers to an amino acid residue at the position recited in a peptide, or an amino acid residue which is similar, identical, or homologous to the residue recited in a peptide. Identifying an amino acid at a corresponding position may be determining a particular amino acid in a sequence that refers to a particular sequence. As used herein, the term "corresponding region" generally refers to a similar or corresponding position in a related protein or reference protein.

For example, based on the alignment of any amino acid sequence with SEQ ID NO: 1, each amino acid residue in the amino acid sequence may be numbered with reference to the position number of the amino acid residue corresponding to the amino acid residue of SEQ ID NO: 1. For example, a sequence alignment algorithm such as that described herein can identify the position of an amino acid or a position where modifications such as substitutions, insertions or deletions occur as compared to a query sequence (also referred to as a "reference sequence").

For such alignments, for example, the Needleman-Wunsch algorithm (Needleman and Wunsch, 1970, J. Mol. Biol. 48: 443-453), the Needleman program of the EMBOSS package (EMBOSS: The European Molecular Biology Open Software Suite, Rice et al., 2000, Trends Genet. 16: 276-277), *etc*. may be used, but are not limited thereto, and sequence alignment programs, pairwise sequence comparison algorithms, *etc*., known in the art may be appropriately used.

Another aspect of the present disclosure provides a polynucleotide encoding the modified aspartate kinase of the present disclosure.

The lysC gene of the present disclosure may include all genes known to encode proteins having LysC activity.

Specifically, the *lysC* gene of the present disclosure may be a polynucleotide encoding NCgl0247 (Cg0306) derived from a microorganism of the genus *Corynebacterium*, but is not limited thereto. In another example, the *lysC* gene may be a *lysC* gene derived from a microorganism of the genus *Corynebacterium*, but is not limited thereto, and it is apparent that *lysC* genes of various origins encoding proteins having LysC protein activity are included.

As used herein, the term "polynucleotide" refers to a DNA or RNA strand having at least a certain length, which is a polymer of nucleotides in which nucleotide monomers are connected in a long chain by covalent bonds. More specifically, it refers to a polynucleotide fragment encoding the modified aspartate kinase.

The polynucleotide encoding the modified aspartate kinase of the present disclosure may comprise a nucleotide sequence encoding a modified aspartate kinase in which the amino acid corresponding to position 250 and/or position 258 in the amino acid sequence of SEQ ID NO: 1 is substituted with another amino acid, or may comprise a nucleotide sequence in which the codon corresponding to position 748 and/or position 772 or 773 in the nucleotide sequence of SEQ ID NO: 2 is substituted with a codon encoding another amino acid. In one example, the polynucleotide of the present disclosure may comprise a nucleotide sequence encoding the amino acid sequence represented by SEQ ID NO: 3, SEQ ID NO: 5, or SEQ ID NO: 7. In a more specific example of the present disclosure, the polynucleotide of the present disclosure may have or comprise the sequence of SEQ ID NO: 4, SEQ ID NO: 6, or SEQ ID NO: 8. Furthermore, the polynucleotide of the present disclosure may consist of or essentially consist of the sequence of SEQ ID NO: 4, SEQ ID NO: 6, or SEQ ID NO: 8.

The polynucleotide of the present disclosure may have various modifications in coding regions within a range that does not change the amino acid sequence of the modified aspartate kinase of the present disclosure, in consideration of degeneracy of codons or codons preferred in an organism in which the modified aspartate kinase of the present disclosure is to be expressed. Specifically, the polynucleotide of the present disclosure may have or comprise a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2, or may consist of or essentially consist of a nucleotide sequence having 70% or more, 75% or more, 80% or more, 85% or more, 90% or more, 95% or more, 96% or more, 97% or more, 98% or more, and less than 100% homology or identity to the sequence of SEQ ID NO: 2, but is not limited thereto. In particular, in a sequence having the homology or identity, the codon encoding the amino acid corresponding to position 250 and/or position 258 of SEQ ID NO: 1 may be one of the codons encoding an amino acid other than valine, for example, methionine, serine, threonine, asparagine, cysteine, histidine, lysine, aspartic acid, alanine, tyrosine, leucine, glutamine, glycine, proline, glutamic acid, arginine, isoleucine, phenylalanine, and tryptophan.

In addition, the polynucleotide of the present disclosure may comprise a probe that can be prepared from a known gene sequence without limitation, for example, any sequence capable of hybridizing with a complementary sequence to all or a part of the polynucleotide sequence of the present disclosure under stringent conditions. The term "stringent condition" refers to a condition that allows specific hybridization between polynucleotides. These conditions are specifically described in the literature (see J. Sambrook et al., Molecular Cloning, A Laboratory Manual, 2nd Edition, Cold Spring Harbor Laboratory press, Cold Spring Harbor, New York, 1989; F.M. Ausubel et al.,Current Protocols in Molecular Biology, John Wiley & Sons, Inc., New York, 9.50-9.51, 11.7-11.8). For example, the stringent conditions may include conditions under which polynucleotides having a high homology or identity of 70% or more, 75% or more, 80% or more, 85% or more, 90% or more, 95% or more, 96% or more, 97% or more, 98% or more, or 99% or more are hybridized with each other and polynucleotides having a homology or identity lower than the above are not hybridized with each other; or common washing conditions of Southern hybridization, that is, washing once, specifically, twice or three times at a salt concentration and a temperature corresponding to 60°C, 1 × SSC, 0.1% SDS, specifically, 60°C, 0.1 × SSC, 0.1% SDS, and more specifically 68°C, 0.1 × SSC, 0.1% SDS.

Hybridization requires that two nucleic acids have complementary sequences, although mismatches between bases may be possible depending on the stringency of hybridization. The term "complementary" is used to describe the relationship between nucleotide bases capable of hybridizing with each other. For example, regarding DNA, adenine is complementary to thymine, and cytosine is complementary to guanine. Therefore, the polynucleotide of the present disclosure may also include an isolated nucleic acid fragment complementary to the entire sequence as well as a nucleic acid sequence substantially similar thereto.

Specifically, polynucleotides having homology or identity to the polynucleotide of the present disclosure may be detected using hybridization conditions comprising a hybridization step at a Tₘ value of 55 °C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length and degree of complementarity of the polynucleotides, and these variables are well known in the art (*e.g.,* Sambrook *et al*., supra).

Still another aspect of the present disclosure provides a vector comprising the polynucleotide of the present disclosure.

The vector may be an expression vector for expressing the polynucleotide in a host cell, but is not limited thereto.

The vector of the present disclosure may comprise a DNA construct comprising the nucleotide sequence of a polynucleotide encoding the target polypeptide operably linked to a suitable expression regulatory region (or expression regulatory sequence) so as to enable expression of the target polypeptide in a suitable host cell. The expression regulatory region may comprise a promoter capable of initiating transcription, any operator sequence for controlling such transcription, a sequence encoding a suitable mRNA ribosome binding site, and a sequence controlling termination of transcription and translation. The vector, after being transformed into a suitable host cell, may replicate or function independently of the host genome, or may be integrated into the genome itself.

The vector used in the present disclosure is not particularly limited, and any vector known in the art may be used. Examples of commonly used vectors include plasmids, cosmids, viruses, and bacteriophages, either in their natural state or in a recombinant state. For example, as a phage vector or cosmid vector, pWE15, M13, MBL3, MBL4, IXII, ASHII, APII, t10, t11, Charon4A, Charon21A, *etc*. may be used; and as a plasmid vector, those based on pDZ, pBR, pUC, pBluescriptII, pGEM, pTZ, pCL, pET, *etc*. may be used. Specifically, vectors such as pDZ, pDC, pDC24, pACYC177, pACYC184, pCL, pECCG117, pUC19, pBR322, pMW118, and pCC1BAC may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. A selection marker for confirming the chromosomal insertion may be further included. The selection marker is used to screen cells transformed with the vector, i.e., to confirm the insertion of the target nucleic acid molecule, and markers that confer selectable phenotypes, such as drug resistance, auxotrophy, resistance to cytotoxic agents, or expression of surface polypeptides, may be used. In an environment treated with a selective agent, only cells expressing the selection marker survive or exhibit a different phenotypic trait, thereby enabling selection of transformed cells.

As used herein, the term "transformation" refers to the introduction of a vector comprising a polynucleotide encoding a target polypeptide into a host cell or a microorganism such that the polypeptide encoded by the polynucleotide can be expressed in the host cell. As long as the transformed polynucleotide can be expressed within the host cell, the transformed polynucleotide may be located within the chromosome of the host cell or exist extrachromosomally. Further, the polynucleotide comprises DNA and/or RNA encoding the polypeptide. The polynucleotide may be introduced in any form, provided that it can be introduced into and expressed within a host cell. For example, the polynucleotide may be introduced into a host cell in the form of an expression cassette, which is a gene construct comprising all elements necessary for self-expression. The expression cassette can usually comprise a promoter operably linked to the polynucleotide, a transcription termination signal, a ribosome binding site, and a translation termination signal. The expression cassette may be in the form of a self-replicable expression vector. Further, the polynucleotide may be introduced into a host cell as-is and operably linked to a sequence necessary for expression in the host cell, but is not limited thereto.

Further, as used herein, the term "operably linked" means that the polynucleotide sequence is functionally linked to a promoter sequence that initiates and mediates transcription of the polynucleotide encoding the target modified aspartate kinase of the present disclosure.

Still another aspect of the present disclosure provides the modified aspartate kinase of the present disclosure or a microorganism comprising a polynucleotide encoding the modified aspartate kinase.

The strain of the present disclosure may comprise the modified aspartate kinase of the present disclosure, a polynucleotide encoding the modified aspartate kinase, or a vector comprising the polynucleotide of the present disclosure.

As used herein, the term "microorganism (or strain)" comprises all wild-type microorganisms or naturally or artificially genetically modified microorganisms, and may be a microorganism in which a particular mechanism is weakened or enhanced due to insertion of a foreign gene, enhancement or inactivation of the activity of an endogenous gene, *etc*., and may be a microorganism comprising genetic modification to produce a target polypeptide, protein, or product.

The strain of the present disclosure may be a strain naturally having an amino acid-producing ability, or a microorganism in which an amino acid-producing ability has been imparted to a strain lacking an amino acid-producing ability. In one example, it may be a microorganism into which the modified aspartate kinase of the present disclosure or a polynucleotide encoding the same has been introduced to increase amino acid-producing ability, but is not limited thereto.

As used herein, the term "amino acid" may include glutamic acid as well as all L-amino acids that can be biosynthesized using glutamic acid as a precursor. In one example, L-amino acids that can be produced through the glutamic acid biosynthetic pathway using glutamic acid as a precursor include glutamine, ornithine, citrulline, arginine, *etc*., and any other L-amino acids that can be biosynthesized using glutamic acid as a precursor may be included in the scope of the present disclosure. In addition, glutamic acid that can be produced with the involvement of the aspartate kinase of the present disclosure and substances synthesized using the same as a precursor may be included without limitation.

Meanwhile, as used herein, the term "L-amino acid" includes both proteinogenic and non-proteinogenic amino acids.

The strain of the present disclosure may be a microorganism having an increased amino acid-producing ability compared to a parent strain not comprising the modified aspartate kinase of the present disclosure or a wild-type strain of the genus *Corynebacterium*. The microorganism may be one in which an amino acid-producing ability has been enhanced by introduction of the modified aspartate kinase of the present disclosure.

As an example, an aspartate kinase-unmodified microorganism serving as a control strain for comparing whether amino acid-producing ability is increased may be *Corynebacterium glutamicum* (*C. glutamicum*) ATCC 13869 strain, C. gl::argF*_argR* strain producing ornithine, C. gl::argR*_argG* strain producing citrulline, or CJR100 strain producing arginine, but is not limited thereto.

In one example, the recombinant strain having increased producing ability may exhibit an increase of at least about 3%, specifically, at least about 3%, at least about 4%, at least about 5%, at least about 6%, at least about 7%, at least about 9%, or at least about 11% (the upper limit is not particularly limited and may be, for example, about 200% or less, about 150% or less, about 100% or less, or about 50% or less) compared to the amino acid-producing ability of a parent strain before modification or a non-modified microorganism. However, it is not limited thereto, as long as it exhibits a positive increase compared to the producing ability of a parent strain before modification, a non-modified microorganism, or an aspartate kinase-unmodified microorganism. In another example, the recombinant strain having an increased amino acid-producing ability may have an amino acid producing ability that is increased by at least about 1.01-fold, at least about 1.05-fold, at least about 1.10-fold, at least about 1.15-fold, at least about 1.20-fold, at least about 1.25-fold, at least about 1.30-fold, at least about 1.325-fold, at least about 1.35-fold, at least about 1.375-fold, at least about 1.40-fold, at least about 1.425-fold, at least about 1.45-fold, at least about 1.46-fold, or at least about 1.47-fold (the upper limit is not particularly limited and may be, for example, about 10-fold or less, about 5-fold or less, about 3-fold or less, or about 2-fold or less) compared to that of a parent strain before modification, a non-modified microorganism, or an aspartate kinase-unmodified microorganism, but is not limited thereto.

As used herein, the term "non-modified microorganism" does not exclude a strain comprising mutations that may occur naturally in a microorganism, and may refer to a wild-type strain or a natural strain *per se*, or a strain before a trait is altered by genetic variation due to natural or artificial factors. Additionally, as used herein, the term "aspartate kinase-unmodified microorganism" may refer to a strain into which the aspartate kinase variant of the present disclosure has not been introduced, or a strain prior to introduction thereof. The aspartate kinase-unmodified microorganism of the present disclosure does not exclude strains comprising modifications of proteins or genes other than modifications of aspartate kinase or a polynucleotide encoding the same.

As used in the present disclosure, the term "unmodified microorganism" may be used interchangeably with "strain before modification", "microorganism before modification", "non-mutant strain", "unmodified strain", "non-mutant microorganism", or "reference microorganism."

The microorganism of the present disclosure may be a microorganism comprising a modified aspartate kinase or a polynucleotide encoding the same; or a microorganism (*e.g.,* a recombinant microorganism) genetically modified to comprise a modified aspartate kinase, a polynucleotide encoding the same, or a vector comprising the polynucleotide, but is not limited thereto. The term "endogenous activity" refers to the activity of a particular polypeptide originally possessed by a parent strain before transformation or a wild-type or non-modified microorganism, when a trait is altered through genetic modification caused by natural or artificial factors. This term may be used interchangeably with "activity before modification".

In another example of the present disclosure, the microorganism of the present disclosure may be *Corynebacterium glutamicum, Corynebacterium stationis, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium pollutisoli, Corynebacterium imitans, Corynebacterium testudinoris*, or *Corynebacterium flavescens*, and specifically *Corynebacterium glutamicum*, but is not limited thereto.

In another example of the present disclosure, the recombinant microorganism of the present disclosure may be a microorganism in which the activity of certain proteins in the amino acid biosynthesis pathway is further enhanced, or the activity of certain proteins in the amino acid degradation pathway is further weakened, thereby increasing the amino acid-producing ability.

In another example of the present disclosure, the recombinant microorganism of the present disclosure may be a strain having increased producing ability of ornithine, which is one of the amino acids.

In an embodiment according to any one of the preceding embodiments, the recombinant microorganism of the present disclosure may be one that has been modified to enhance the activity of one or more selected from the group consisting of acetylglutamate synthase that converts glutamate to N-acetylglutamate, ornithine acetyltransferase (ArgJ) that converts acetylornithine to ornithine, acetylglutamate kinase (ArgB) that converts acetylglutamate to N-acetylglutamyl phosphate, acetyl gamma glutamyl phosphate reductase (ArgC) that converts acetylglutamyl phosphate to N-acetylglutamate semialdehyde, and acetylornithine aminotransferase (ArgD) that converts acetylglutamate semialdehyde to N-acetylornithine, relative to the endogenous activity, thereby improving ornithine-producing ability.

However, it is not limited thereto, and ornithine-producing ability can be enhanced by gene expression regulation methods known in the art.

In another example of the present disclosure, the recombinant microorganism of the present disclosure may be a strain having increased producing ability of citrulline, which is one of the amino acids.

In an embodiment according to any one of the preceding embodiments, the recombinant microorganism of the present disclosure may be one that has been modified to enhance the activity of ornithine carbamoyltransferase (ArgF) that converts ornithine to citrulline, relative to the endogenous activity, thereby improving citrulline-producing ability. Alternatively, the recombinant microorganism of the present disclosure may be one in which citrulline-producing ability is improved as a result of the improved producing ability of ornithine, which is a precursor of citrulline, as described above.

However, it is not limited thereto, and citrulline-producing ability can be enhanced by gene expression regulation methods known in the art.

In another example of the present disclosure, the recombinant microorganism of the present disclosure may be a strain having increased producing ability of arginine, which is one of the amino acids.

In an embodiment according to any one of the preceding embodiments, the recombinant microorganism of the present disclosure may be one that has been modified to enhance the activity of one or more selected from the group consisting of argininosuccinate synthase (ArgG), argininosuccinate lyase (ArgH), aspartate ammonia lyase (AspA), and aspartate aminotransferase (AspB), relative to the endogenous activity, thereby improving arginine-producing ability. Alternatively, the recombinant microorganism of the present disclosure may be one in which arginine-producing ability is improved as a result of the improved producing ability of ornithine, which is a precursor of citrulline, as described above.

However, it is not limited thereto, and arginine-producing ability can be enhanced by gene expression regulation methods known in the art.

As used herein, the term "weakening" of the activity of a polypeptide (including, for example, proteins specified by the names of respective enzymes) is a concept including both having reduced activity or having no activity compared to the endogenous activity. The weakening may be used interchangeably with terms such as inactivation, deficiency, down-regulation, decrease, reduction, and attenuation.

The weakening may also include a case where the activity of the polypeptide itself is reduced or removed compared to the activity of the polypeptide originally possessed by the microorganism due to mutation of a polynucleotide encoding the polypeptide, *etc*.; a case where the overall intracellular polypeptide activity level and/or concentration (expression level) is lower compared to a natural strain due to the inhibition of expression of the gene of a polynucleotide encoding the polypeptide, or the inhibition of translation into the polypeptide, *etc*.; a case where the polynucleotide is not expressed at all; and/or a case where no polypeptide activity is observed even when the polynucleotide is expressed. The expression that the polypeptide activity is "inactivated", "deficient", "decreased", "down-regulated", "reduced", or "attenuated" compared to the endogenous activity means that the polypeptide activity is decreased compared to the activity of a particular polypeptide originally possessed by a parent strain before transformation or a non-modified microorganism.

Such weakening of polypeptide activity can be performed by any method known in the art, but the method is not limited thereto, and can be achieved by applying various methods well known in the art (*e.g.,* Nakashima N et al., Bacterial cellular engineering by genome editing and gene silencing. Int J Mol Sci. 2014;15(2):2773-2793, Sambrook et al. Molecular Cloning 2012, *etc*.).

Specifically, the weakening of the polypeptide of the present disclosure may result from:
1) deleting a part or all of a gene encoding a polypeptide;
2) modifying an expression regulatory region (or expression regulatory sequence) such that expression of a gene encoding a polypeptide is decreased;
3) modifying an amino acid sequence (*e.g.,* deletion/substitution/addition of at least one amino acid in the amino acid sequence) constituting a polypeptide such that the polypeptide activity is removed or weakened;
4) modifying a gene sequence encoding a polypeptide such that the polypeptide activity is removed or weakened (*e.g.,* deletion/substitution/addition of at least one nucleobase in a nucleobase sequence of a polypeptide gene to encode a polypeptide that has been modified to remove or weaken the polypeptide activity);
5) modifying a nucleotide sequence encoding the initiation codon or 5'-UTR of a gene transcript encoding a polypeptide;
6) introducing an antisense oligonucleotide (*e.g.,* antisense RNA) that complementarily binds to the gene transcript encoding a polypeptide;
7) adding a sequence complementary to a Shine-Dalgarno (SD) sequence of a gene encoding a polypeptide at the front end of the SD sequence to form a secondary structure incapable of ribosomal attachment;
8) reverse transcription engineering (RTE), which adds a reversely transcribed promoter to the 3' end of an open reading frame (ORF) of a gene sequence encoding a polypeptide; or
9) regulating the cellular localization of a protein (polypeptide); or
10) a combination of at least two selected from 1) to 9) above, but is not particularly limited thereto.

For example,
1) Deleting a part or all of the gene encoding a polypeptide may be deleting all of the polynucleotide encoding an endogenous target polypeptide within the chromosome, replacing with a polynucleotide in which some nucleotides are deleted, or replacing with a marker gene.

In addition, 2) modifying an expression regulatory region (or expression regulatory sequence) may be inducing a modification on the expression regulatory region (or expression regulatory sequence) through deletion, insertion, non-conservative substitution or conservative substitution, or a combination thereof; or replacing the sequence with a sequence having a weaker activity. The expression regulatory region comprises a promoter, an operator sequence, a sequence encoding a ribosome binding site, and a sequence for regulating the termination of transcription and translation, but is not limited thereto.

Modifying an amino acid sequence or a polynucleotide sequence of 3) and 4) may be inducing a modification on the amino acid sequence of the polypeptide or the polynucleotide sequence encoding the polypeptide through deletion, insertion, non-conservative or conservative substitution, or a combination thereof in the sequence to weaken the activity of the polypeptide; or replacing with an amino acid sequence or a polynucleotide sequence modified to have a weaker activity, or an amino acid sequence or a polynucleotide sequence modified to have no activity, but are not limited thereto. For example, the expression of a gene may be inhibited or weakened by introducing a mutation into the polynucleotide sequence to form a termination codon, but is not limited thereto.

5) Modifying a nucleotide sequence encoding an initiation codon or 5'-UTR of a gene transcript encoding a polypeptide may be, for example, substituting it with a nucleotide sequence encoding another initiation codon having a lower polypeptide expression rate than the endogenous initiation codon, but is not limited thereto.

6) Introducing an antisense oligonucleotide (*e.g.,* antisense RNA) that complementarily binds to the gene transcript encoding a polypeptide, can be found in the literature [Weintraub, H. et al., Antisense-RNA as a molecular tool for genetic analysis, Reviews - Trends in Genetics, Vol. 1(1) 1986].

7) Adding a sequence complementary to a Shine-Dalgarno (SD) sequence of a gene encoding a polypeptide at the front end of the SD sequence to form a secondary structure incapable of ribosomal attachment may be inhibiting mRNA translation or reducing the speed thereof.

8) Reverse transcription engineering (RTE), which adds a reversely transcribed promoter to the 3' end of an open reading frame (ORF) of a gene sequence encoding a polypeptide, may be forming an antisense nucleotide complementary to the gene transcript encoding a polypeptide to weaken the activity thereof.

The 9) regulating the cellular localization of a protein (polypeptide) may be targeting the protein (polypeptide) to a specific intracellular organelle or to a specific intracellular space. For example, it may be targeting the protein (polypeptide) to the periplasm or cytoplasm through addition or removal of a leader sequence that functions in protein targeting, but is not limited thereto.

As used herein, the term "enhancement" of the activity of a polypeptide means that the activity of the polypeptide is increased compared to the endogenous activity. The enhancement may be used interchangeably with terms such as activation, up-regulation, overexpression, and increase. Herein, the activation, enhancement, up-regulation, overexpression, and increase may include both exhibition of an activity not originally possessed, or exhibition of an improved activity compared to the endogenous activity or activity before modification. The "enhancement", "up-regulation", "overexpression", or "increase" in the activity of a polypeptide compared to the endogenous activity means that the activity and/or concentration (expression level) of the polypeptide is enhanced compared to that of a particular polypeptide originally possessed by a parent strain before transformation or a non-modified microorganism.

The enhancement may be achieved by introducing a foreign polypeptide, or by enhancing the activity and/or concentration (expression level) of the endogenous polypeptide. The enhancement of the activity of a polypeptide can be confirmed by the increase in the level of activity or expression level of the polypeptide, or by the amount of product excreted from the polypeptide.

The enhancement of the activity of a polypeptide can employ various methods well known in the art, and the method is not limited as long as it can enhance the activity of the target polypeptide compared to that of a microorganism before modification. Specifically, genetic engineering and/or protein engineering well known to those skilled in the art as common methods in molecular biology may be used, but the method is not limited thereto (*e.g.,* Sitnicka et al., Functional Analysis of Genes. Advances in Cell Biology. 2010, Vol. 2. 1-16, Sambrook et al. Molecular Cloning 2012, *etc*.).

Specifically, the enhancement of the polypeptide of the present disclosure may result from:
1) increasing the intracellular copy number of a polynucleotide encoding a polypeptide;
2) replacing an expression regulatory region of a gene encoding a polypeptide on the chromosome with a sequence having a stronger activity;
3) modifying a nucleotide sequence encoding the initiation codon or 5'-UTR of a gene transcript encoding a polypeptide;
4) modifying the amino acid sequence of a polypeptide such that the activity of the polypeptide is enhanced;
5) modifying a polynucleotide sequence encoding a polypeptide such that the activity of the polypeptide is enhanced (*e.g.,* modifying a polynucleotide sequence of a polypeptide gene to encode a polypeptide that has been modified to enhance the activity of the polypeptide);
6) introducing a foreign polypeptide exhibiting polypeptide activity or a foreign polynucleotide encoding the same;
7) codon-optimization of a polynucleotide encoding a polypeptide;
8) analyzing the tertiary structure of a polypeptide then selecting and modifying an exposed site, or chemically modifying the same;
9) regulating the cellular localization of a protein (polypeptide); or
10) a combination of at least two selected from 1) to 9) above, but is not particularly limited thereto.

More specifically,
1) Increasing the intracellular copy number of a polynucleotide encoding a polypeptide may be achieved by introducing into a host cell a vector operably linked to the polynucleotide encoding the polypeptide and is able to replicate and function regardless of the host. Alternatively, it may be achieved by introducing one copy or at least two copies of the polynucleotide encoding the polypeptide into the chromosome of a host cell. The introduction into the chromosome may be performed by introducing into the host cell a vector capable of inserting the polynucleotide into the chromosome of the host cell, but is not limited thereto. The vector is as described above.
2) Replacing an expression regulatory region (or expression regulatory sequence) of a gene encoding a polypeptide on the chromosome with a sequence having a strong activity may be, for example, inducing a modification on the sequence through deletion, insertion, non-conservative or conservative substitution, or a combination thereof to further enhance the activity of the expression regulatory region, or replacing the sequence with a sequence having a stronger activity. The expression regulatory region may comprise, but is not particularly limited to, a promoter, an operator sequence, a sequence encoding a ribosome binding site, a sequence regulating the termination of transcription and translation, *etc*. In one example, it may be replacing the original promoter with a strong promoter, but is not limited thereto.
   Examples of known strong promoters include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, *etc*., but the strong promoter is not limited thereto.
3) Modifying a nucleotide sequence encoding the initiation codon or 5'-UTR of a gene transcript encoding a polypeptide may, for example, substituting with a nucleotide sequence encoding another initiation codon having a higher expression rate of the polypeptide compared to the endogenous initiation codon, but is not limited thereto.

Modifying an amino acid sequence or a polynucleotide sequence of 4) and 5) may be inducing a modification on the amino acid sequence of the polypeptide or the polynucleotide sequence encoding the polypeptide through deletion, insertion, non-conservative or conservative substitution, or a combination thereof in the sequence to enhance the activity of the polypeptide; or replacing it with an amino acid sequence or a polynucleotide sequence modified to have a stronger activity, or an amino acid sequence or a polynucleotide sequence modified to have enhanced activity, but are not limited thereto. The replacement may specifically be performed by inserting the polynucleotide into a chromosome by homologous recombination, but is not limited thereto. The vector used herein may further comprise a selection marker for confirming the insertion into a chromosome.

6) Introducing a foreign polynucleotide exhibiting polypeptide activity may be introducing into a host cell a foreign polynucleotide encoding a polypeptide that exhibits the same or similar activity to that of the polypeptide. The foreign polynucleotide is not limited by its origin or sequence, as long as it exhibits the same/similar activity to that of the polypeptide. The introduction may be performed by a transformation method known in the art appropriately selected by those skilled in the art, and the expression of the introduced polynucleotide in the host cell enables the production of the polypeptide, thereby increasing its activity.

The 7) codon-optimization of a polynucleotide encoding a polypeptide may be codon-optimization of an endogenous polynucleotide to increase the transcription or translation within a host cell, or codon-optimization of the foreign polynucleotide such that optimal transcription and translation can be achieved within the host cell.

8) Analyzing the tertiary structure of a polypeptide then selecting and modifying an exposed site, or chemically modifying the same may be, for example, comparing the sequence information of a polypeptide to be analyzed with a database storing sequence information of known proteins to determine template protein candidates according to the degree of sequence similarity, and thus confirming the structure based on the information to select and transform or modify the exposed site to be modified or chemically modified.

The 9) regulating the cellular localization of a protein (polypeptide) may be targeting the protein (polypeptide) to a specific intracellular organelle or to a specific intracellular space. For example, it may be targeting the protein (polypeptide) to the periplasm or cytoplasm through addition or removal of a leader sequence that functions in protein targeting, but is not limited thereto.

Such enhancement of polypeptide activity may be an increase in the activity or concentration expression level of the corresponding polypeptide relative to the activity or concentration of the polypeptide expressed in a wild-type microbial strain or a microbial strain before modification, or an increase in the amount of product produced from the polypeptide, but is not limited thereto.

The modification of a part or all of a polynucleotide in the microorganism of the present disclosure may be induced by (a) homologous recombination using a vector for chromosomal insertion in the microorganism or genome editing using an engineered nuclease (*e.g.,* CRISPR-Cas9), and/or (b) light, such as ultraviolet rays and radiation, *etc*. and/or chemical treatments, but is not limited thereto. The method of modifying a part or all of the gene may include a method using DNA recombination technology. For example, a part or all of the gene may be deleted by injecting a nucleotide sequence or a vector comprising a nucleotide sequence homologous to a target gene into the microorganism to induce homologous recombination. The injected nucleotide sequence or vector may comprise a dominant selection marker, but is not limited thereto.

In the microorganism of the present disclosure, the modified aspartate kinase, polynucleotide, amino acid, *etc*. are as described in the other aspects above.

Still another aspect of the present disclosure provides a method for producing an amino acid comprising culturing, in a medium, a microorganism comprising the modified aspartate kinase of the present disclosure or a polynucleotide encoding the modified aspartate kinase.

The method for producing an amino acid of the present disclosure may comprise culturing, in a medium, a microorganism comprising the modified aspartate kinase of the present disclosure, the polynucleotide of the present disclosure, or the vector of the present disclosure.

As used herein, the term "culturing" refers to growing the microorganism of the present disclosure under properly controlled environmental conditions. The culturing process of the present disclosure may be performed in a suitable medium known in the art under suitable culturing conditions known in the art. Such a culturing process may be easily adjusted and used by those skilled in the art according to the selected microorganism. Specifically, the culturing may be batch culturing, continuous culturing, and/or fed-batch culturing, but is not limited thereto.

As used herein, the term "medium" refers to a mixed substance containing nutrients required to culture the microorganism of the present disclosure as a main component, and the medium supplies nutrients, growth factors, *etc*., including water, which are indispensable for survival and development. Specifically, any medium and culture conditions may be used for culturing the microorganism of the present disclosure without particular limitation, as long as the medium is used for the common culture of microorganisms. The microorganism of the present disclosure may be cultured in a common medium that contains suitable carbon sources, nitrogen sources, phosphorus sources, inorganic compounds, amino acids, and/or vitamins, *etc*., while controlling the temperature, pH, *etc*. under aerobic conditions.

Specifically, the culture medium for the microorganism of the genus *Corynebacterium* can be found in the literature ["Manual of Methods for General Bacteriology" by the American Society for Bacteriology (Washington D.C., USA, 1981)].

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (i.e., molasses converted into reducing sugars) may be used, and other various carbon sources in appropriate amounts may be used without limitation. These carbon sources may be used alone or in combination of two or more thereof, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extracts, yeast extract, malt extract, corn steep liquor, casein hydrolysate, fish or decomposition products thereof, and skim soybean cake or decomposition products thereof may be used. These nitrogen sources may be used alone or in combination of two or more thereof, but are not limited thereto.

As the phosphorus sources, monopotassium phosphate, dipotassium phosphate, or sodium-containing salts corresponding thereto may be used. As for the inorganic compounds, sodium chloride, calcium chloride, iron chloride, magnesium sulfate, iron sulfate, manganese sulfate, calcium carbonate, *etc*. may be used. Additionally, amino acids, vitamins, and/or appropriate precursors, *etc*. may be included. These components or precursors may be added to the medium in a batchwise or continuous manner. However, they are not limited thereto.

During the culturing of the microorganism of the present disclosure, the pH of the medium may be adjusted by adding a compound such as ammonium hydroxide, potassium hydroxide, ammonia, phosphoric acid, or sulfuric acid to the medium in an appropriate manner. Further, during the culturing, foaming may be suppressed by using an antifoaming agent such as fatty acid polyglycol ester. To maintain an aerobic state of the medium, oxygen or oxygen-containing gas may be injected into the medium. To maintain an anaerobic or microaerobic state of the medium, no gas may be injected, or nitrogen, hydrogen, or carbon dioxide gas may be injected. However, the culturing conditions are not limited thereto.

In the culturing of the present disclosure, the culturing temperature may be maintained at 20°C to 45°C, specifically 25°C to 40°C, and the culturing may be performed for about 10 to 160 hours, but the culturing conditions are not limited thereto.

Amino acids produced by the culturing of the present disclosure may be secreted into the medium or remain in the cells. In particular, the amino acid is as described above.

The method for producing an amino acid of the present disclosure may further comprise preparing the microorganism of the present disclosure, preparing a medium for culturing the strain, or a combination thereof (in any order), for example, prior to the culturing.

The method for producing an amino acid of the present disclosure may further comprise recovering amino acid from the culture medium (medium in which the culture was grown) or the *Corynebacterium glutamicum* strain. The recovering step may be further included after the culturing.

The recovery may be collecting the target amino acid using a suitable method known in the art according to the culturing method of the microorganism of the present disclosure, for example, a batch, continuous, fed-batch culturing method, *etc*. For example, centrifugation, filtration, treatment with a crystallized protein precipitating agent (salting-out), extraction, sonication, ultrafiltration, dialysis, various types of chromatography such as molecular sieve chromatography (gel filtration), adsorption chromatography, ion exchange chromatography, affinity chromatography, HPLC, and a combination of these methods may be used, and the target amino acid can be recovered from the medium or the microorganism using suitable methods known in the art.

Further, the method for producing an amino acid of the present disclosure may further comprise purification. The purification may be performed using a suitable method known in the art. In one example, when the method for producing an amino acid of the present disclosure comprises both recovery and purification, the recovery and purification may be performed continuously or non-continuously regardless of the order, or integrated into a simultaneous or a single step, but the method is not limited thereto.

In the method of the present disclosure, the modified aspartate kinase, polynucleotide, vector, strain, amino acid, *etc*. are as described in the other aspects above.

Still another aspect of the present disclosure provides a composition for producing an amino acid, comprising: the modified aspartate kinase of the present disclosure; a polynucleotide encoding the modified aspartate kinase; a microorganism comprising the modified aspartate kinase of the present disclosure or the polynucleotide encoding the modified aspartate kinase; a culture of the microorganism; or a combination of two or more thereof.

The composition of the present disclosure may further comprise any suitable excipient commonly used in compositions for producing amino acids, and such excipients may be, for example, preservatives, wetting agents, dispersing agents, suspending agents, buffers, stabilizers, isotonic agents, *etc*., but is not limited thereto.

Still another aspect of the present disclosure provides use of the modified aspartate kinase of the present disclosure; a polynucleotide encoding the modified aspartate kinase; or a microorganism comprising the modified aspartate kinase of the present disclosure or the polynucleotide encoding the modified aspartate kinase, for amino acid production.

The aspartate kinase, modified aspartate kinase, polynucleotide, vector, strain, medium, amino acid, *etc*. are as described in the other aspects above.

### [Mode for Carrying Out the Invention]

The present disclosure will be described in detail by way of Examples. However, these Examples are given for illustrative purposes only, and the scope of the present disclosure is not intended to be limited by these Examples. Meanwhile, technical descriptions absent in the present disclosure may be sufficiently understood and readily practiced by those skilled in the art of the present disclosure or related art.

### Example 1: Construction of Recombinant Vector for Introduction of Modified Aspartate Kinase

Vectors for introducing V250L, V258I, and V250L+V258I mutations into the *lysC* gene encoding an aspartate kinase in wild-type *Corynebacterium glutamicum* were constructed.

To construct the vectors for introducing each mutation, the genome of wild-type *Corynebacterium glutamicum* ATCC 13869 was used as a template to amplify the homologous recombinant A arm and homologous recombinant B arm. The primer pairs for each mutation are as shown in Table 1 below.

**[Table 1]**

| Name | Homologous recombinant A arm (5'→3') | Homologous recombinant B arm (5'→3') |
|---|---|---|
| V250L | SEQ ID NOs: 9 and 10 | SEQ ID NOs: 11 and 12 |
| V258I | SEQ ID NOs: 9 and 13 | SEQ ID NOs: 12 and 14 |
| V250L+V258I | SEQ ID NOs: 9 and 15 | SEQ ID NOs: 12 and 16 |

The PCR was performed under conditions of denaturation at 95°C for 5 minutes, followed by 30 cycles of denaturation at 95°C for 30 seconds, annealing at 55°C for 30 seconds, and polymerization at 72°C for 1 minute, and then polymerization at 72°C for 5 minutes. Subsequently, PCR fragments were extracted using a gel purification kit (QIAGEN). The homologous recombinant A arm and B arm gene fragments obtained above and the vector pDC24 (SEQ ID NO: 19) digested with BamHI and SalI restriction enzymes were ligated by the Gibson assembly method (DG Gibson et al., NATURE METHODS, VOL.6 NO.5, MAY 2009, NEBuilder HiFi DNA Assembly Master Mix). Each construct was transformed into *E. coli* DH5α and plated on LB solid medium containing 25 mg/L kanamycin. To select transformant colonies, PCR was performed using the primer pair of SEQ ID NO: 17 and SEQ ID NO: 18. Plasmids were obtained from selected colonies using a commonly known plasmid extraction method, and the obtained plasmids were named pDC24-lysC(V250L), pDC24-lysC(V258I), and pDC24-lysC(V250L+V258I), respectively.

The primer sequences used herein are shown in Table 2 below.

**[Table 2]**

| SEQ ID NO | Name | Sequence (5'→3') |
|---|---|---|
| 9 | primer 1 | TCGGTACCCGGGGATCCGTGGCCCTGGTCGTACAG |
| 10 | primer 2 | ACTGCTTCTTCCAAAGGAATATCCTCCATAGAGCC |
| 11 | primer 3 | CCTTTGGAAGAAGCAGTCCTTACCGGTATCGCAAC |
| 12 | primer 4 | CATGCCTGCAGGTCGACCAGAGAACAACGCAACGTC |
| 13 | primer 5 | TTGTCGGTTGCGATACCGGTAAGGACTGCTTCTTCC |
| 14 | primer 6 | GGTATCGCAACCGACAAGTCCGAAGCCAAAGTAAC |
| 15 | primer 7 | GGTAAGGACTGCTTCTTCCAAAGGAATATCCTCCATAG |
| 16 | primer 8 | AGAAGCAGTCCTTACCGGTATCGCAACCGACAAGTCC |
| 17 | primer 9 | TATTACGCCAGCTGGCGAAA |
| 18 | primer 10 | GCTTTACACTTTATGCTTCC |

### Example 2: Evaluation of L-Citrulline-Producing Ability

### Example 2-1. Construction of Control Group

To construct a control strain for the evaluation of L-citrulline-producing ability, a vector for substituting the glutamic acid at position 47 of the protein sequence of argR (ANU33619.1) with a stop codon was constructed. Using the genome of wild-type *C. glutamicum* ATCC 13869 as a template, the homologous recombinant A arm was amplified using the primer pair of SEQ ID NOs: 20 and 21, and the homologous recombinant B arm was amplified using the primer pair of SEQ ID NOs: 22 and 23. A plasmid was then obtained by the same method as in Example 1, and this plasmid was named pDC24-argR(E47*).

Additionally, a vector for substituting the phenylalanine at position 68 of the protein sequence of argG (ANU33620.1) with a stop codon was constructed. Using the genome of *C. glutamicum* ATCC 13869 as a template, the homologous recombinant A arm was amplified using the primers of SEQ ID NOs: 24 and 25, and the homologous recombinant B arm was amplified using the primers of SEQ ID NOs: 26 and 27. A plasmid was then obtained by the same method as in Example 1, and this plasmid was named pDC24-argG(F68*).

The primer sequences used herein are shown in Table 3 below.

**[Table 3]**

| SEQ ID NO | Name | Sequence (5'→3') |
|---|---|---|
| 20 | primer 11 | CGGTACCCGGGGATCCCTCGTGCGGAATTCGTGGAG |
| 21 | primer 12 | ATCCAGCAGCAATTCAGACA |
| 22 | primer 13 | CTGAATTGCTGCTGGATTAAGGCATCGATATCACCCA |
| 23 | primer 14 | ATGCCTGCAGGTCGACCCTTCATTTTAAGTTCCTTG |
| 24 | primer 15 | CGGTACCCGGGGATCCTTCATCGATAGGGTGGG |
| 25 | primer 16 | GTACTCCTCAGCTTACTCATCCTTTGCATCAACA |
| 26 | primer 17 | AGTAAGCTGAGGAGTACTGCCTGCCAACCATCAA |
| 27 | primer 18 | ATGCCTGCAGGTCGACCGACTGGCTTGCCACCCT |

Using the constructed pDC24-argR(E47*) vector, wild-type *C. glutamicum* ATCC 13869 was transformed by electroporation (Appl. Microbiol. Biotechnol. (1999) 52:541-545), and through a second crossover process, a strain was obtained in which position 139 of the nucleotide sequence of argR was substituted from guanine (G) to thymine (T), resulting in substitution of position 47 of the protein sequence with a stop codon. PCR and nucleotide sequence analysis were performed using the primer pair of SEQ ID NOs: 20 and 23, which can amplify the region adjacent to and including the insertion site of the gene, and the genetic modification was confirmed. The microorganism thus obtained was named C. gl::argR*.

To additionally delete argG from the obtained C. gl::argR*, transformation was performed in the same manner using the pDC24-argG(F68*) vector to obtain a microorganism. PCR and nucleotide sequence analysis were performed using the primer pair of SEQ ID NOs: 24 and 27, which can amplify the region adjacent to and including the insertion site of the gene, and the genetic modification was confirmed. The microorganism thus obtained was named C. gl::argR*_argG*

### Example 2-2. Construction of Strain Expressing Modified Aspartate Kinase

To construct mutant strains with mutations introduced into the aspartate kinase based on the C. gl::argR*_argG* strain obtained in Example 2-1, the C. gl::argR*_argG* strain was transformed by electroporation using pDC24-lysC(V250L), pDC24-lysC(V258I), and pDC24-lysC(V250L+V258I) of Example 1, and through a second crossover process, mutant strains were obtained in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively. A DNA fragment comprising the chromosomal *lysC* gene was PCR-amplified from the genome of the obtained strain using the primer pair of SEQ ID NOs: 9 and 12. The PCR was performed under conditions of denaturation at 95°C for 10 minutes, followed by 30 cycles of denaturation at 95°C for 30 seconds, annealing at 55°C for 30 seconds, and polymerization at 72°C for 2 minutes, and then polymerization at 72°C for 10 minutes. Analysis of the nucleotide sequences of the amplified genes confirmed that mutations were introduced into the nucleotide sequences encoding the 250th and/or 258th amino acids downstream from the ORF start codon of the *lysC* gene in each strain (Table 4).

**[Table 4]**

| Amino Acid (Nucleotide Sequence) Before Substitution | Amino Acid (Nucleotide Sequence) After Substitution |
|---|---|
| 250th Val (GTG) | Leu (TTG) |
| 258th Val (GTC) | Ile (ATC) |

Accordingly, based on the C. gl::argR*_argG* strain, mutant strains were obtained in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively, and the obtained mutant strains were named C. gl::argR*_argG*_lysC(V250L), C. gl::argR*_argG*_lysC(V258I), and C. gl::argR*_argG*_lysC(V250L+V258I), respectively.

### Example 2-3. Evaluation of Citrulline-Producing Ability

The C. gl::argR*_argG*_lysC(V250L), C. gl::argR*_argG*_lysC(V258I), and C. gl::argR*_argG*_lysC(V250L+V258I) strains of Example 2-2 and the control strain C. gl::argR*_argG* were cultured using the production medium described below to compare L-citrulline-producing abilities. Each strain was inoculated into a 250 mL corner-baffled flask containing 25 mL of production medium and shake-cultured at 200 rpm at 33°C for 48 hours. After completion of the culture, the concentration of citrulline was measured by HPLC. The results are shown in Table 5 below.

### <Production Medium>

Raw sugar 50 g, (NH₄)₂SO₄ 30 g, Yeast extract 1 g, KH₂PO₄ 1.1 g, MgSO₄·7H₂O 1.2 g, L-arginine 0.2 g, Biotin 1 mg, Thiamine HCl 5 mg, Calcium pantothenate 5 mg, Nicotinamide 15 mg, MnSO₄ 10 mg, FeSO₄ 10 mg, ZnSO₄ 0.5 mg, CuSO₄ 0.5 mg, CaCO₃ 30 g, pH 7.2 (based on 1 L of distilled water)

**[Table 5]**

| Strain | L-Citrulline Concentration (g/L) | Percentage (%) |
|---|---|---|
| C. gl::argR*_argG* | 3.07 | 100 |
| C. gl::argR*_argG*_lysC(V250L) | 3.35 | 109 |
| C. gl::argR*_argG*_lysC(V258I) | 3.29 | 107 |
| C. gl::argR*_argG*_lysC(V250L+V258I) | 3.42 | 111 |

As a result, as shown in Table 5 above, all of the mutant strains in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively, exhibited increased citrulline production and yield compared to the control strain C. gl::argR*_argG*.

### Example 3: Evaluation of L-Ornithine-Producing Ability

### Example 3-1. Construction of Control Group

To construct a control strain for the evaluation of L-ornithine-producing ability, a vector for substituting the serine at position 55 of the protein sequence of ArgF (ANU33618.1) with a stop codon was constructed. Using the genome of wild-type *C. glutamicum* ATCC 13869 as a template, the homologous recombinant A arm was amplified using the primer pair of SEQ ID NOs: 28 and 29, and the homologous recombinant B arm was amplified using the primer pair of SEQ ID NOs: 30 and 31. A plasmid was then obtained by the same method as in Example 1, and was named pDC24-argF(S55*).

Additionally, a vector for substituting the glutamic acid at position 47 of the protein sequence of ArgR (ANU33619.1) with a stop codon was constructed. Using the genome of wild-type *C. glutamicum* ATCC 13869 as a template, the homologous recombinant A arm was amplified using the primer pair of SEQ ID NOs: 20 and 21, and the homologous recombinant B arm was amplified using the primer pair of SEQ ID NOs: 22 and 23. A plasmid was then obtained by the same method as in Example 1, and this plasmid was named pDC24-argR(E47*).

The primer sequences used herein are shown in Table 6 below.

**[Table 6]**

| SEQ ID NO | Name | Sequence (5'→3') |
|---|---|---|
| 20 | primer 11 | CGGTACCCGGGGATCCCTCGTGCGGAATTCGTGGAG |
| 21 | primer 12 | ATCCAGCAGCAATTCAGACA |
| 22 | primer 13 | CTGAATTGCTGCTGGATTAAGGCATCGATATCACCCA |
| 23 | primer 14 | ATGCCTGCAGGTCGACCCTTCATTTTAAGTTCCTTG |
| 28 | primer 19 | CGGTACCCGGGGATCCTGACCCCAGGCAAGCACGG |
| 29 | primer 20 | GAAGCGAGTACGAGTTTAAGTCTTATC |
| 30 | primer 21 | AAACTCGTACTCGCTTCTCC |
| 31 | primer 22 | ATGCCTGCAGGTCGACCGGCGCCGGCAACCTCGTC |

Using the constructed pDC24-argF(S55*) vector, wild-type *C. glutamicum* ATCC 13869 was transformed by electroporation (Appl. Microbiol. Biotechnol. (1999) 52:541-545), and through a second crossover process, a microorganism was obtained in which position 164 of the nucleotide sequence of argF was substituted from cytosine (C) to adenine (A), resulting in substitution of position 55 of the protein sequence with a stop codon. PCR and nucleotide sequence analysis were performed using the primer pair of SEQ ID NOs: 28 and 31, which can amplify the region adjacent to and including the insertion site of the gene, and the genetic modification was confirmed. The microorganism thus obtained was named C. gl::argF*.

To additionally delete argR from the obtained C. gl::argF*, the same transformation was performed using the pDC24-argR(E47*) vector to obtain a microorganism. PCR and nucleotide sequence analysis were performed using the primer pair of SEQ ID NOs: 20 and 23, which can amplify the region adjacent to and including the insertion site of the gene, and the genetic modification of argR deletion was confirmed. The microorganism thus obtained was named C. gl::argR*_argF*.

### Example 3-2. Construction of Strain Expressing Modified Aspartate Kinase

To construct mutant strains with mutations introduced into the aspartate kinase based on the C. gl::argR*_argF* strain obtained in Example 3-1, the C. gl::argR*_argF* strain was transformed by electroporation using pDC24-lysC(V250L), pDC24-lysC(V258I), and pDC24-lysC(V250L+V258I) of Example 1, and through a second crossover process, mutant strains were obtained in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively. A DNA fragment comprising the chromosomal lysC gene was PCR-amplified from the genome of the obtained strain using the primer pair of SEQ ID NOs: 9 and 12. The PCR was performed under conditions of denaturation at 95°C for 10 minutes, followed by 30 cycles of denaturation at 95°C for 30 seconds, annealing at 55°C for 30 seconds, and polymerization at 72°C for 2 minutes, and then polymerization at 72°C for 10 minutes. Analysis of the nucleotide sequences of the amplified genes confirmed that mutations were introduced into the nucleotide sequences encoding the 250th and 258th amino acids downstream from the ORF start codon of the *lysC* gene in each strain (Table 4).

Accordingly, based on the C. gl::argR*_argF* strain, mutant strains were obtained in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively, and the obtained mutant strains were named C. gl::argR*_argF*_lysC(V250L), C. gl::argR*_argF*_lysC(V258I), and C. gl::argR*_argF*_lysC(V250L+V258I), respectively.

### Example 3-3. Evaluation of Ornithine-Producing Ability

The C. gl::argR*_argF*_lysC(V250L), C. gl::argR*_argF*_lysC(V258I), and C. gl::argR*_argF*_lysC(V250L+V258I) strains of Example 3-2 and the control strain C. gl::argR*_argF* were cultured using the production medium described below to compare L-ornithine-producing abilities. Each strain was inoculated into a 250 mL corner-baffled flask containing 25 mL of production medium and shake-cultured at 200 rpm at 32°C for 48 hours. After completion of the culture, the concentration of ornithine was measured by HPLC. The results are shown in Table 7 below.

### <Production Medium>

Raw sugar 50 g, (NH₄)₂SO₄ 30 g, Yeast extract 1 g, KH₂PO₄ 1.1 g, MgSO₄·7H₂O 1.2 g, L-arginine 0.2 g, Biotin 1 mg, Thiamine HCl 5 mg, Calcium pantothenate 5 mg, Nicotinamide 15 mg, MnSO₄ 10 mg, FeSO₄ 10 mg, ZnSO₄ 0.5 mg, CuSO₄ 0.5 mg, CaCO₃ 30 g, pH 7.2 (based on 1 L of distilled water)

**[Table 7]**

| Strain | L-Ornithine Amount Produced (g/L) | Percentage (%) |
|---|---|---|
| C. gl::argR*_argF* | 15.1 | 100 |
| C. gl::argR*_argF*_lysC(V250L) | 15.8 | 105 |
| C. gl::argR*_argF*_lysC(V258I) | 15.5 | 103 |
| C. gl::argR*_argF*_lysC(V250L+V258I) | 15.7 | 104 |

As a result, as shown in Table 9 above, all of the mutant strains in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively, exhibited increased ornithine production and yield compared to the control strain C. gl::argR*_argF*.

### Example 4: Evaluation of L-Arginine-Producing Ability

### Example 4-1. Construction of Control Group

To evaluate L-arginine-producing ability, the *Corynebacterium glutamicum* strain CJR2 was constructed by introducing ΔargR and argB(M54V) mutations into wild-type *Corynebacterium glutamicum* ATCC13869 (Ikeda, Masato et al., Applied and environmental microbiology 75(6):1635-41, 2009). First, argR deletion and argB(M54V) mutation introduction vectors were constructed. Using the genomic DNA of *Corynebacterium glutamicum* ATCC13869 as a template, PCR was performed using the primer pairs of SEQ ID NOs: 32 and 33, and SEQ ID NOs: 34 and 35, followed by overlapping PCR using the primer pair of SEQ ID NOs: 32 and 35, to obtain a homologous recombination fragment having an argR deletion mutation sequence. To prepare a homologous recombination fragment having the argB(M54V) mutation by the same method, PCR was performed using the primer pairs of SEQ ID NOs: 36 and 37, and SEQ ID NOs: 38 and 39, followed by overlapping PCR using SEQ ID NOs: 36 and 39. The PCR was performed under conditions of denaturation at 95°C for 5 minutes, followed by 28 cycles of denaturation at 95°C for 30 seconds, annealing at 55°C for 30 seconds, and polymerization at 72°C for 2 minutes, and then polymerization at 72°C for 5 minutes. The fragments obtained above were purified and then fusion-cloned with the pDC24 vector (SEQ ID NO: 19) digested with SmaI restriction enzyme using the In-Fusion^{®} HD Cloning Kit (Clontech) according to the manual to obtain plasmids. The constructed vectors were named pDC24-ΔargR and pDC24-argB(M54V), respectively.

Next, an argR deletion mutation was introduced into wild-type *Corynebacterium glutamicum* ATCC13869. Transformation was performed by electroporation using the constructed pDC24-ΔargR plasmid (van der Rest et al., Appl Microbiol Biotechnol 52:541-545, 1999). A second recombination was then performed on a solid plate medium comprising 4% sucrose, and PCR was performed as described above on the second recombination-completed transformant using the primer pair of SEQ ID NOs: 32 and 35. It was confirmed that a deletion mutation was introduced into the chromosomal *argR* gene. The transformant was named CJR1.

### <Solid Plate Medium (pH 7.0)>

Glucose 10 g, Peptone 10 g, Beef extract 5 g, Yeast extract 5 g, Brain Heart Infusion 18.5 g, NaCl 2.5 g, Urea 2 g, Sorbitol 91 g, Agar 20 g (based on 1 L of distilled water)

The argB(M54V) mutation was introduced into *Corynebacterium glutamicum* CJR1 by the same method as described above. The constructed pDC24-argB(M54V) plasmid was used, and PCR was performed on the second recombination-completed transformant using the primer pair of SEQ ID NOs: 36 and 39 to confirm that the M54V mutation was introduced into the chromosomal *argB* gene, and the transformant was named CJR2.

Based on the constructed CJR2 strain, the CJR100 strain with enhanced N-acetyl-gamma-glutamyl-phosphate reductase (hereinafter referred to as argC) gene was constructed.

To enhance the activity of argC (NCBI accession number BBD29_RS07530), which is an N-acetyl-gamma-glutamyl-phosphate reductase, a plasmid was constructed to enhance argC activity by replacing the wild-type promoter of the *argC* gene with Po2 (US 10273491 B) known as a strong promoter. Specifically, to construct a strain into which argC with the Po2 promoter is introduced, PCR was performed using the chromosomal DNA of *Corynebacterium glutamicum* ATCC13869 as a template to amplify the upstream region of the *argC* gene using the primers of SEQ ID NOs: 40 and 41, and to amplify a gene fragment of the downstream region of the *argC* gene using the primers of SEQ ID NOs: 42 and 43, respectively. Additionally, a Po2 promoter fragment was obtained using SEQ ID NOs: 44 and 45, with the synthesized Po2 promoter as a template. Pfu Ultra^{™} high-fidelity DNA polymerase (Stratagene) was used as the polymerase for the PCR reaction, and the PCR was performed under conditions of denaturation at 95°C for 5 minutes, followed by 28 cycles of denaturation at 95°C for 30 seconds, annealing at 55°C for 30 seconds, and polymerization at 72°C for 1 minute, and then polymerization at 72°C for 5 minutes. As a result, an 86 bp DNA fragment of the Po2 promoter region, a 610 bp DNA fragment of the upstream region of *Corynebacterium glutamicum* ATCC13869 argC, and a 1086 bp DNA fragment of the downstream region thereof were obtained, respectively. PCR was performed using the primers of SEQ ID NOs: 40 and 43 with the amplified promoter and DNA fragments as templates. The PCR was performed under conditions of denaturation at 95°C for 5 minutes, followed by 28 cycles of denaturation at 95°C for 30 seconds, annealing at 55°C for 30 seconds, and polymerization at 72°C for 2 minutes, and then polymerization at 72°C for 5 minutes. The two fragments obtained above underwent DNA purification and then were linked by fusion cloning with the pDC24 plasmid digested with SmaI restriction enzyme using the In-Fusion^{®} HD Cloning Kit (Clontech). The vector thus obtained was named pDC24-Po2-argC.

The CJR2 strain constructed above was then transformed by electroporation using the constructed pDC24-Po2-argC plasmid (van der Rest et al., Appl Microbiol Biotechnol 52:541-545, 1999). A second recombination was then performed on a solid plate medium comprising 4% sucrose, and PCR was performed on the second recombination-completed transformant using the primers of SEQ ID NOs: 40 and 45. It was confirmed that that the chromosomal *argC* gene was enhanced with the Po2 promoter. In particular, the PCR reaction was performed by the same method as described above, and the transformant thus obtained was named CJR100.

The primer sequences used herein are shown in Table 8 below.

**[Table 8]**

| SEQ ID NO | Name | Sequence (5'→3') |
|---|---|---|
| 32 | primer 23 | TGAATTCGAGCTCGGTACCCCACTGGTGAACTCCTTGTCC |
| 33 | primer 24 | TTGAACTAGGGGCGCTTTAAAAGTTTTCCGGTGTTGACGG |
| 34 | primer 25 | CCGTCAACACCGGAAAACTTTTAAAGCGCCCCTAGTTCAA |
| 35 | primer 26 | GTCGACTCTAGAGGATCCCCCGTTGAACTGCTTGCCAGCC |
| 36 | primer 27 | TGAATTCGAGCTCGGTACCCTGCGGCTCGCACGGTTGCTC |
| 37 | primer 28 | ACGGTGCGCAAGAAGACCACGTCGGCAGCAAAAGCAGCCT |
| 38 | primer 29 | GGCTGCTTTTGCTGCCGACGTGGTCTTCTTGCGCACCGTG |
| 39 | primer 30 | GTCGACTCTAGAGGATCCCCCTCTTATCAGGCCAATCGGT |
| 40 | primer 31 | GTGAATTCGAGCTCGGTACCCGCCCCGAAAAGCCGTTAAAAG |
| 41 | primer 32 | TGCCAAAATTCACGATTATTGCCCACCTACAGCTAAAACTGC |
| 42 | primer 33 | TTATTGGAGGAGATCAAAACAATGACAATCAAGGTTGCAATC |
| 43 | primer 34 | CAGGTCGGCGTCGCACCTTAAGGGGATCCTCTAGAGTCGACC |
| 44 | primer 35 | CAATAATCGTGAATTTTGGCA |
| 45 | primer 36 | TGTTTTGATCTCCTCCAATAA |

### Example 4-2. Construction of Strain Expressing Modified Aspartate Kinase

To construct mutant strains with point mutations introduced into the aspartate kinase based on the CJR100 strain obtained in Example 4-1, the CJR100 strain was transformed by electroporation using pDC24-lysC(V250L), pDC24-lysC(V258I), and pDC24-lysC(V250L+V258I) of Example 1, and through a second crossover process, mutant strains were obtained in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively. A DNA fragment comprising the chromosomal *lysC* gene was PCR-amplified from the genome of the obtained strain using the primer pair of SEQ ID NOs: 9 and 12. The PCR was performed under conditions of denaturation at 95°C for 10 minutes, followed by 30 cycles of denaturation at 95°C for 30 seconds, annealing at 55°C for 30 seconds, and polymerization at 72°C for 2 minutes, and then polymerization at 72°C for 10 minutes. Analysis of the nucleotide sequences of the amplified genes confirmed that mutations were introduced into the nucleotide sequences encoding the 250th and 258th amino acids downstream from the ORF start codon of the *lysC* gene in each strain (Table 4).

Accordingly, based on the CJR100 strain, mutant strains were obtained in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively, and the obtained mutant strains were named CJR100_lysC(V250L), CJR100_lysC(V258I), and CJR100_lysC(V250L+V258I), respectively.

### Example 4-3. Evaluation of Arginine-Producing Ability

The CJR100_lysC(V250L), CJR100_lysC(V258I), and CJR100_lysC(V250L+V258I) strains of Example 4-2 and the control strain CJR100 were cultured using the production medium described below to compare L-arginine-producing abilities. Each strain was inoculated into a 250 mL corner-baffled flask containing 25 mL of production medium and shake-cultured at 200 rpm at 32°C for 48 hours. After completion of the culture, the concentration of arginine was measured by HPLC. The results are shown in Table 9 below.

### <Production Medium>

Raw sugar 50 g, (NH₄)₂SO₄ 30 g, Yeast extract 1 g, KH₂PO₄ 1.1 g, MgSO₄·7H₂O 1.2 g, L-arginine 0.2 g, Biotin 1 mg, Thiamine HCl 5 mg, Calcium pantothenate 5 mg, Nicotinamide 15 mg, MnSO₄ 10 mg, FeSO₄ 10 mg, ZnSO₄ 0.5 mg, CuSO₄ 0.5 mg, CaCO₃ 30 g, pH 7.2 (based on 1 L of distilled water)

**[Table 9]**

| Strain Name | L-Arginine Concentration (g/L) | Percentage (%) |
|---|---|---|
| CJR100 | 5.90 | 100 |
| CJR100_lysC(V250L) | 6.23 | 106 |
| CJR100_lysC(V258I) | 6.17 | 105 |
| CJR100_lysC(V250L+V258I) | 6.21 | 105 |

As a result, as shown in Table 11 above, all of the mutant strains in which, from the N-terminus of aspartate kinase, the 250th amino acid valine was substituted with leucine, the 258th amino acid valine was substituted with isoleucine, or both the 250th and the 258th amino acids were simultaneously substituted with leucine and isoleucine, respectively, exhibited increased arginine production and yield compared to the control strain CJR100.

As set forth above, those skilled in the art will be able to understand that the present disclosure may be embodied in other specific forms without departing from the technical spirit or essential characteristics thereof. In this regard, the embodiments described above should be understood to be illustrative rather than restrictive in every respect. The scope of the present disclosure should be construed as comprising the meaning and scope of the appended claims rather than the detailed description, and all changes or variations derived from equivalent concepts fall within the scope of the present disclosure.

## Claims

1. A modified aspartate kinase, wherein an amino acid corresponding to position 250 and/or position 258 in the amino acid sequence of SEQ ID NO: 1 is substituted with another amino acid.

2. The modified aspartate kinase according to claim 1, wherein the amino acid corresponding to position 250 in the amino acid sequence of SEQ ID NO: 1 is substituted with leucine;
the amino acid corresponding to position 258 in the amino acid sequence of SEQ ID NO: 1 is substituted with isoleucine; or
a combination thereof.

3. The modified aspartate kinase according to claim 1, wherein the modified aspartate kinase has a sequence identity of 80% or more to the amino acid sequence of SEQ ID NO: 1.

4. The modified aspartate kinase according to claim 1, wherein the modified aspartate kinase comprises the amino acid sequence of SEQ ID NO: 3, SEQ ID NO: 5, or SEQ ID NO: 7.

5. A polynucleotide encoding the modified aspartate kinase of any one of claims 1 to 4.

6. A microorganism comprising a modified aspartate kinase, wherein an amino acid corresponding to position 250 and/or position 258 in the amino acid sequence of SEQ ID NO: 1 is substituted with another amino acid; or a polynucleotide encoding the modified aspartate kinase.

7. The microorganism according to claim 6, wherein the microorganism has an increased amino acid-producing ability as compared to a microorganism comprising a wild-type aspartate kinase having the amino acid sequence of SEQ ID NO: 1, or a polynucleotide encoding the wild-type aspartate kinase.

8. The microorganism according to claim 7, wherein the amino acid is one or more selected from the group consisting of glutamic acid, citrulline, glutamine, ornithine, and arginine.

9. The microorganism according to claim 6, wherein the microorganism is of the genus *Corynebacterium*.

10. The microorganism according to claim 9, wherein the microorganism of the genus *Corynebacterium* is *Corynebacterium glutamicum*.

11. A method for producing an amino acid, comprising culturing, in a medium, a microorganism comprising a modified aspartate kinase, wherein an amino acid corresponding to position 250 and/or position 258 in the amino acid sequence of SEQ ID NO: 1 is substituted with another amino acid; or a polynucleotide encoding the modified aspartate kinase.

12. The method according to claim 11, wherein the amino acid is one or more selected from the group consisting of glutamic acid, citrulline, glutamine, ornithine, and arginine.

13. The method according to claim 11, further comprising recovering the amino acid from the cultured microorganism, a culture of the microorganism, a fermentation product of the microorganism, or the culture medium.

14. A composition for producing an amino acid, comprising: the modified aspartate kinase of any one of claims 1 to 4; a polynucleotide encoding the modified aspartate kinase; a microorganism comprising the modified aspartate kinase or the polynucleotide encoding the modified aspartate kinase; a culture of the microorganism; or a combination of two or more thereof.

15. Use of the modified aspartate kinase of any one of claims 1 to 4; a polynucleotide encoding the modified aspartate kinase; or a microorganism comprising the modified aspartate kinase or the polynucleotide encoding the modified aspartate kinase, for amino acid production.
